# EUROPEAN PATENT APPLICATION

(11) **EP 2 829 535 A1**
(43) Date of publication of application: **28.01.2015**
(21) Application number: 13178053.8
(22) Date of filing: 25.07.2013
(51) Int. Cl.: C07D 215/48, C07D 217/26, C07D 241/44

(54) **Intercalating amino acids**

(71) Applicant: Universität Konstanz, 78464 Konstanz (DE)
(72) Inventor: Summerer, Daniel, 78462 Konstanz (DE); Schmidt, Moritz Johannes, 78464 Konstanz (DE)
(74) Representative: Elbel, Michaela

(57) **Abstract**

The invention relates to a non-canonical amino acid (ncAA) comprising a lysine and an aromatic residue and to a peptide comprising such a ncAA. The invention further relates to the use of a ncAA comprising a lysine and an aromatic residue for enhancing the affinity of a nucleic acid binding peptide and to a method for producing a nucleic acid binding compound.

SEQUENCE LISTING

<110>  Universität Konstanz

<120>  Intercalating Amino Acids

<130>  T30049

<160>  30

<170>  PatentIn version 3.3

<210>  1
<211>  9
<212>  PRT
<213>  artificial

<220>
<223>  modified peptide


<220>
<221>  X
<222>  (7)..(7)
<223>  non-canonical amino acis

<400>  1

Gly Cys Leu Arg Phe Gly Xaa Ser Cys
1               5


<210>  2
<211>  18
<212>  PRT
<213>  artificial

<220>
<223>  modified TAT minimal motif


<220>
<221>  X
<222>  (6)..(6)
<223>  non-canonical amino acid

<400>  2

Met Gly Cys Tyr Gly Xaa Lys Lys Arg Arg Gln Arg Arg Arg Cys Gly
1               5                   10                  15


Gly Ser



<210>  3
<211>  454
<212>  PRT
<213>  Methanosarcina mazei

<400>  3

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
            180                 185                 190

Ser Gln Thr Asp Arg Leu Glu Val Leu Leu Asn Pro Lys Asp Glu Ile
        195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Tyr Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Cys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Tyr
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Ile Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  4
<211>  11
<212>  PRT
<213>  artificial

<220>
<223>  modified HIV TAT peptide

<220>

<221>  X
<222>  (3)..(3)
<223>  non-canonical amino acid

<400>  4

Tyr Gly Xaa Lys Lys Arg Arg Gln Arg Arg Arg
1               5                   10


<210>  5
<211>  454
<212>  PRT
<213>  artificial

<220>
<223>  modified PylRS

<400>  5

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Cys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Ala Asp Arg Glu Trp Gly Ile Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  6
<211>  454
<212>  PRT
<213>  artificial

<220>
<223>  modified PylRS

<400>  6

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Cys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Ala Arg Glu Trp Gly Ile Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  7
<211>  40
<212>  DNA
<213>  artificial

<220>
<223>  primer

<400>  7
atcgacctgc tgaaaaaagc gggctgtagc agcatcaaag                              40

<210>  8
<211>  41
<212>  DNA
<213>  artificial

<220>
<223>  primer

<400>  8
gcaccagaac tttgatgctg ctacagcccg cttttttcag c                           41

<210>  9
<211>  33
<212>  DNA
<213>  artificial

<220>
<223>  primer

<400>  9
atctccttac atgcaccatt ccttgcggcg gcg                                     33

<210>  10
<211>  36
<212>  DNA
<213>  artificial

<220>
<223>  primer

<400>  10
gcaaggaatg gtacatgcaa ggagatggcg cccaac                                  36

<210>  11
<211>  36
<212>  DNA
<213>  artificial

<220>
<223>  primer

<400>  11
ttttttctag aatcaatcat ccccataatc cttgtt                                36


<210>  12
<211>  35
<212>  DNA
<213>  artificial

<220>
<223>  primer

<400>  12
tttttgcatg caggcagtta ttggtgccct taaac                                35


<210>  13
<211>  39
<212>  DNA
<213>  artificial

<220>
<223>  primer

<400>  13
cgaaatcagc ctgaactccg gcaaaccgtt tcgtgaact                            39


<210>  14
<211>  40
<212>  DNA
<213>  artificial

<220>
<223>  primer

<400>  14
aaacggtttg ccggagttca ggctgatttc gtctttcgga                           40


<210>  15
<211>  43
<212>  DNA
<213>  artificial

<220>
<223>  primer

<400>  15
cgccctatgc tagcaccaaa tctgggtaac tatctgcgca aac                       43


<210>  16
<211>  43
<212>  DNA
<213>  artificial

<220>
<223>  primer

<400>  16
gcacggtcca gtttgcgcag atagttaccc agatttggtg cta                          43


<210>  17
<211>  33
<212>  DNA
<213>  artificial

<220>
<223>  primer

<400>  17
ttttccatgg gcgataaaat tattcacctg act                                    33


<210>  18
<211>  39
<212>  DNA
<213>  artificial

<220>
<223>  primer

<400>  18
ttttgcggcc gctcaatgat gatgatgatg atgcttgtc                              39


<210>  19
<211>  44
<212>  DNA
<213>  artificial

<220>
<223>  primer

<400>  19
cgaaatatgg catctagggt atcccgactc tgctgctgtt caaa                        44


<210>  20
<211>  40
<212>  DNA
<213>  artificial

<220>
<223>  primer

<400>  20
cagcagagtc gggataccct agatgccata tttcggcgca                             40


<210>  21
<211>  53
<212>  DNA
<213>  artificial

<220>
<223>  primer

<400>  21
tttccatggg ctgtctgcgt tttggctaga gctgcggcgg tagcgctagc ttt              53

<210>  22
<211>  18
<212>  DNA
<213>  artificial

<220>
<223>  primer

<400>  22
aaagctagcg ctaccgcc                                                    18


<210>  23
<211>  38
<212>  DNA
<213>  artificial

<220>
<223>  primer

<400>  23
tttttagatc tatgccagca ctgacaaaat cccaaacc                              38


<210>  24
<211>  48
<212>  DNA
<213>  artificial

<220>
<223>  primer

<400>  24
tttttgtcga ctcaatgatg atgatgatga tgcaggttcg tagagatc                   48


<210>  25
<211>  36
<212>  DNA
<213>  artificial

<220>
<223>  primer

<400>  25
gttggaccaa ttccggccga ccgtgagtgg ggtatc                                36


<210>  26
<211>  38
<212>  DNA
<213>  artificial

<220>
<223>  primer

<400>  26
ccccactcac ggtcggccgg aattggtcca acaacggc                              38


<210>  27
<211>  36

<212>  DNA
<213>  artificial

<220>
<223>  primer

<400>  27
ggaccaattc cgctggcccg tgagtggggt atcgac                                36


<210>  28
<211>  36
<212>  DNA
<213>  artificial

<220>
<223>  primer

<400>  28
ccccactcac gggccagcgg aattggtcca acaacg                                36


<210>  29
<211>  75
<212>  DNA
<213>  artificial

<220>
<223>  primer

<400>  29
cgatttttcc atgggctgct atggctagaa aaaacgccgc cagcgccgcc gctgcggcgg      60

ctcggctagc ttttt                                                       75


<210>  30
<211>  17
<212>  DNA
<213>  artificial

<220>
<223>  primer

<400>  30
aaaaagctag ccgagcc                                                     17

## Description

### Field of the invention

The invention relates to a non-canonical amino acid (ncAA) comprising a lysine and an aromatic residue and to a peptide comprising such a ncAA. The invention further relates to the use of a ncAA comprising a lysine and an aromatic residue for enhancing the affinity of a nucleic acid binding peptide and to a method for producing a nucleic acid binding compound.

### Background of the invention

Nucleic acid binding compounds are used in many different technological fields, in particular life science research and analytical examination. For example, they are used for labeling, isolating and purifying nucleic acid molecules. In addition, nucleic acid binding compounds often interfere with endogenous ligand-DNA interactions, thereby inhibiting cellular processes as e.g. DNA replication or cell signaling pathways. Accordingly, nucleic acid binding compounds are also used for medical applications, e.g. for treating bacterial and viral infections or as anti-cancer drugs.

The interaction of nucleic acid binding molecules with DNA or RNA can occur randomly, e.g. through chemical intercalation or targeted, as for example through DNA binding motifs, which mediate binding to specific DNA or RNA sequences or structures. Random intercalation is often observed for small chemical molecules, which are easy to apply, but also show unspecific binding affinities. Targeted binding, in contrast, is found for DNA binding proteins, which are, due to their large size and precise three dimensional folding, elaborate to produce and difficult to apply, in particular for medical uses.

In summary, the number of molecules, which show reliable and efficient nucleic acid binding, and are likewise easy to apply, in particular for medical use, is limited. Moreover, it would be desirable to target given peptides or proteins, which are known to have specific functions, to DNA or RNA. Thus, there is a need for tools to specifically provide molecules with strong and specific nucleic acid binding properties.

### Summary

In a first aspect, the invention relates to a non-canonical amino acid (ncAA) comprising a lysine and an aromatic residue, wherein the aromatic residue is bound to the N-epsilon atom of the lysine and comprises at least two aromatic rings.

In a further aspect, the invention relates to a ncAA selected from the group consisting of

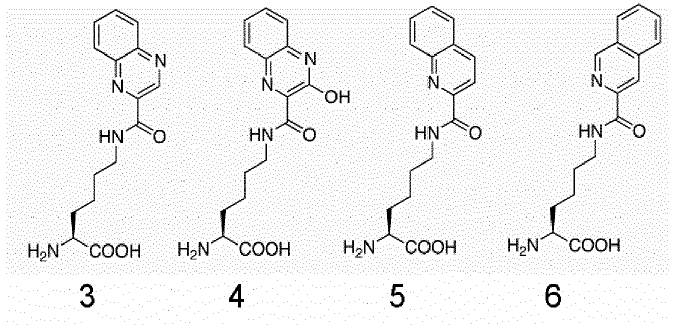

In a further aspect, the invention relates to a peptide comprising a ncAA of the invention.

In a further aspect, the invention relates to a method for producing a nucleic acid-binding compound comprising the steps of providing a nucleic acid sequence encoding for a peptide, introducing a stop codon or a quadruplet codon into the sequence, expressing the sequence in the presence of a tRNA^{Pyl}, a modified pyrrolysyl-tRNA-synthetase (PyIRS), and a ncAA of the invention, which is encoded by the stop codon or the quadruplet codon, wherein the aromatic residue is an nucleic acid intercalating residue.

In a further aspect, the invention relates to a kit comprising a ncAA of the invention, a nucleic acid encoding tRNA^{Pyl}, and a nucleic acid encoding a modified PylRS.

In a further aspect, the invention relates to a use of a ncAA comprising a lysine and an aromatic residue for producing a peptide, wherein the aromatic residue is bound to the N-epsilon atom of the lysine and comprises at least two aromatic rings.

In a further aspect, the invention relates to a modified PylRS having a substitution of alanine or glycine for tyrosine at position 306, wherein the PylRS further comprises a substitution of alanine or glycine for lysine at position 407 and/or a substitution of alanine or glycine for aspartic acid at position 408.

In a further aspect, the invention relates to a nucleic acid encoding for a PylRS of the invention.

### Brief description of the drawings

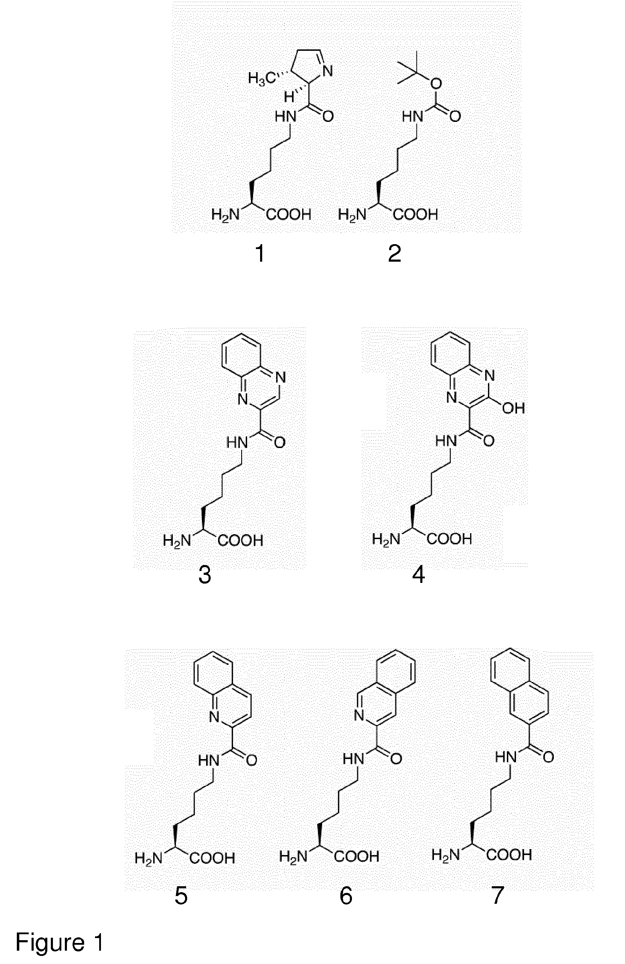

Figure 1 shows the chemical formulas of L-pyrrolysine (1), Nε-tert-butyl-L-lysine (Boc-Lys) (2) and five further non-canonical amino acids comprising a quinoline-isoquinoline or quinoxalinecarboxyamide (ncAA 3 - 7).

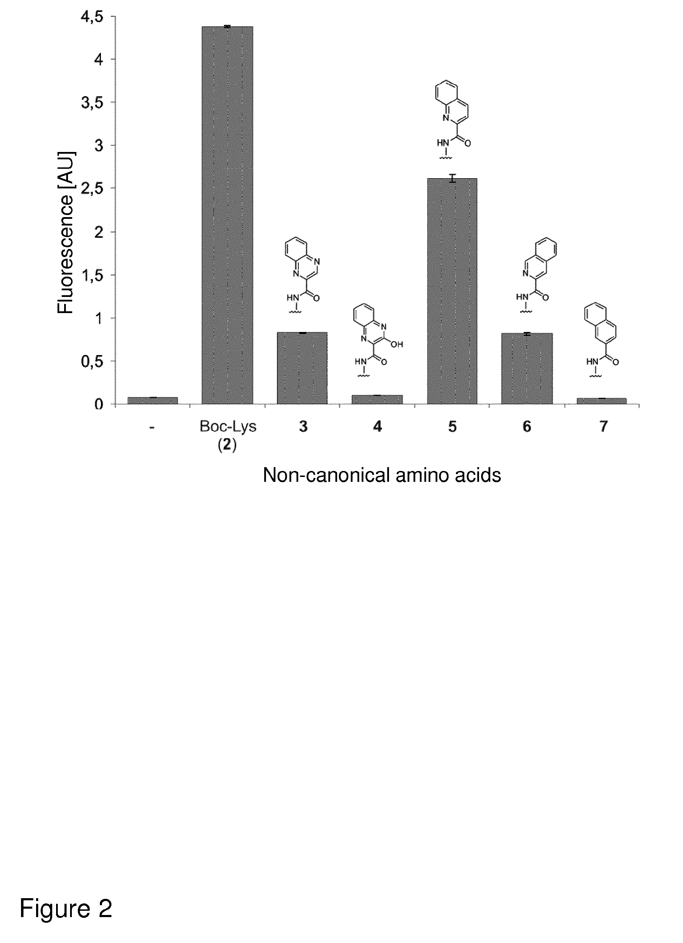

Figure 2 shows the efficiency of PylRS_AF-mediated amber suppression in GFPY39TAG with ncAA 2 - 8 analyzed by whole cell fluorescence measurements. AU = arbitrary units.

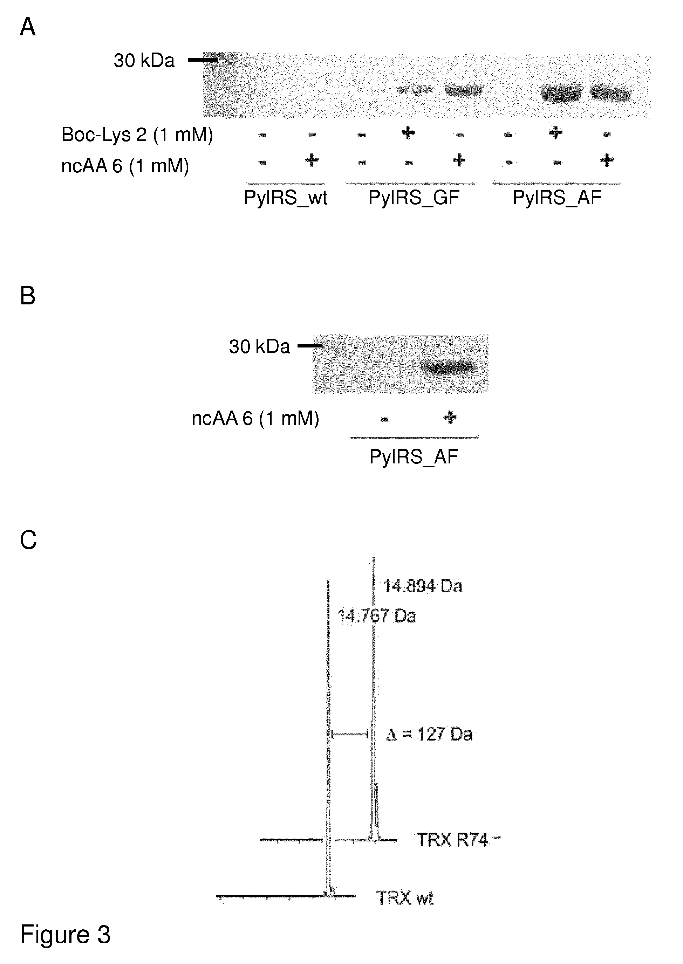

Figure 3 shows the efficient and precise incorporation of quinolinecarboxamide-bearing ncAA 6 of **Figure 1** into proteins in *E. coli.* (A) SDS PAGE analysis of purified GFP depicting the expression of GFPY39→2 and GFP-Y39→6 under co-expression of PylRS_wt, PylRS_GF or PylRS_AF. (B) SDS PAGE analysis of purified TRX-R74→6 expression under co-expression of PylRS_AF. (C) Electrospray ionization mass spectrometry (ESI MS) spectra of purified TRX-wt and TRX-R74→6.

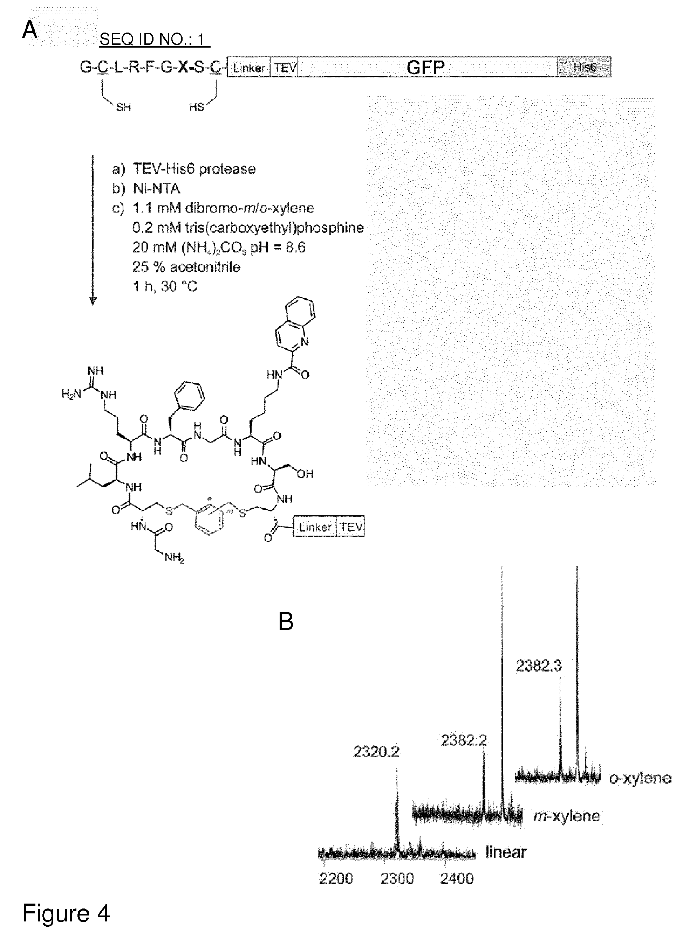

Figure 4 shows the incorporation of quinolinecarboxamide-bearing ncAA 6 into cyclized peptides. (A) Schematic view, cleavage and cyclization of peptide (SEQ ID NO.: 1) construct containing ncAA 6 (marked as X). (B) MALDI-TOF MS spectra of purified, TEV-cleaved linear peptide and cyclization products using either α,α'-dibromo-m- or o-xylene.

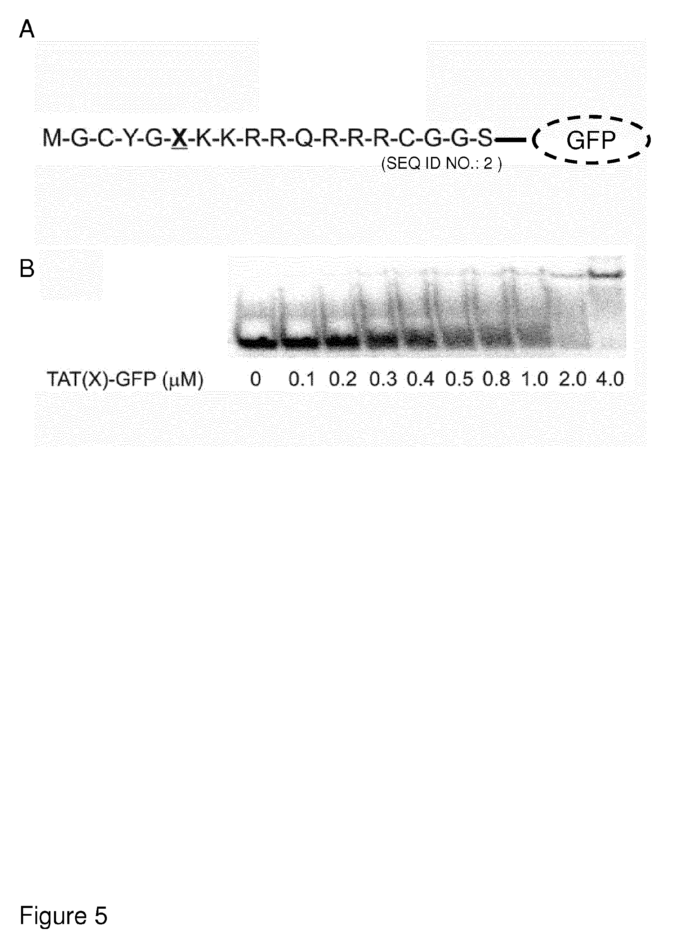

Figure 5 shows the incorporation of quinolinecarboxamide-bearing ncAA 6 of **Figure 1** into the arginine-rich motif of HIV-1 TAT. (A) Schematic view of the GFP fusion construct with N-terminal TAT minimal arginine-rich motif (SEQ ID NO.: 2) containing ncAA 6 (marked as X). (B) Electromobility shift assay (EMSA) showing binding of GFP-TAT construct containing ncAA 6 to TAR RNA with varying concentrations of GFP-TAT construct.

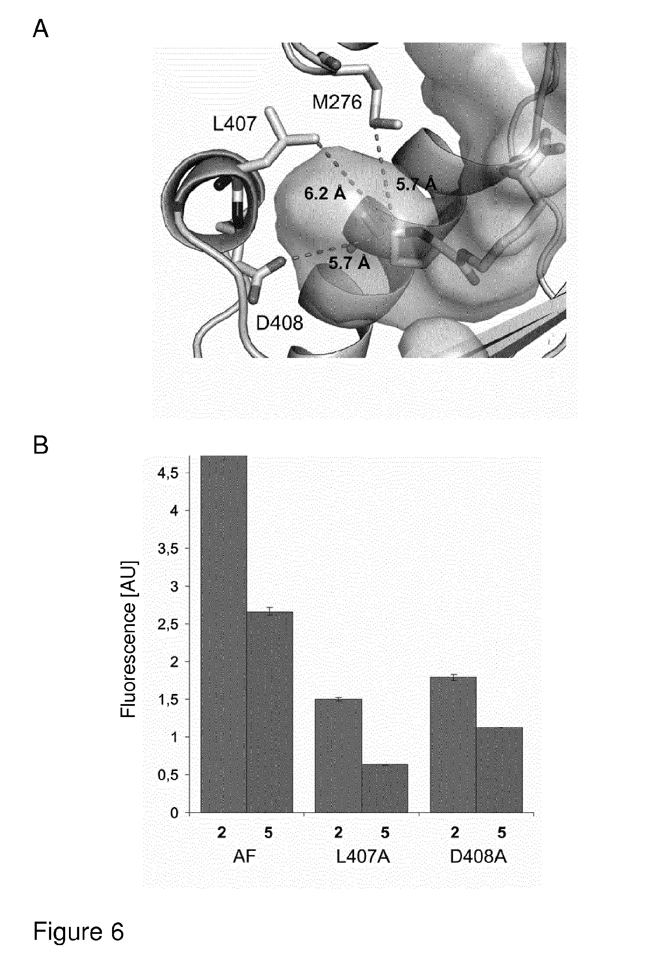

Figure 6 shows the further enlargement of the binding pocket of PylRS_AF. (A) Crystal structure of PylRS_wt in complex with pyrrolysine ncAA 1 and AMPPNP (pdb entry 2ZCE) superimposed with the obtained structure of PylRS_AF in complex with AMPPNP. Shown is only PylRS_AF and Pyrrolysine. The amino acid binding pocket is shown as semi-transparent surface with ncAA 1 and newly exposed amino acids M276, L407 and D408 (that form the new rear part of the pocket) shown as sticks. Distances between C5 of the pyrroline ring of ncAA 1 and closest atoms of M276, L407 and D408 are shown as dotted lines. (B) Efficiency of amber suppression in GFP-Y39TAG with Boc-Lys 2 and quinolinecarboxamide-bearing ncAA 5 catalyzed by PylRS_AF, PylRS_AF L407A and PylRS_AF D408A analyzed by whole cell fluorescence measurements. AU = arbitrary units.

### Detailed description of the invention

In a first aspect, the invention relates to a non-canonical amino acid (ncAA) comprising a lysine and an aromatic residue, wherein the aromatic residue is bound to the N-epsilon atom of the lysine and comprises at least two aromatic rings.

The ncAA of the invention combines two different properties. First, it is a derivative of a natural amino acid, namely lysine, such that it can form an integral part of a peptide or protein without disturbing the molecule's structure. Second, it comprises an aromatic residue, which has at least two aromatic rings and, thus, has the ability to interact with nucleic acid molecules by stacking and / or intercalation.

Since the ncAA has the basic structure of a natural amino acid, i.e. lysine, it can be introduced into a peptide by common methods of protein synthesis. Henceforth, the term "peptide" as used herein refers to a compound produced by amide formation between a carboxyl group of one amino acid and an amino group of another. The term comprises oligopeptides, polypeptides as well as proteins. The peptides may preferably comprise between 5 to 50 amino acids, more preferred 5 to 20 amino acids. The ncAA may be incorporated into a peptide by cellular or cell-free protein synthesis through the use of an orthogonal tRNA/aminoacyl-tRNA-synthetase (aaRS) pair. For example, the ncAA may be incorporated into a peptide by using *Escherichia coli* cells expressing a mutant pyrrolysyl-tRNA-synthetase, which harbors a Y384F and a Y306A mutation (PylRS_AF) (Yanagisawa et al., 2008). Due to said mutations, the PylRS_AF has a ncAA binding pocket, which is considerably enlarged compared to that of wilde type PylRS, and can accommodate the ncAA. Therefore, the modified PylRS is suitable to transfer much larger amino acids compare to wild type PylRS. Using a modified PylRS with an enlarged ncAA binding pocket, functional GFP proteins comprising a quinolinecarboxamide-bearing lysine were obtained **(****Figure 2****).**

By incorporating the ncAA into a peptide, the resulting molecule comprises the aromatic residue of the ncAA. The term "aromatic residue" as used herein refers to a chemical group derived from a structure having a cyclic, delocalized (4n + 2) PI-electron system (according to theory of Hückel; IUPAC), in particular to chromophores. Such residues include arenes and their substitution products such as benzene, naphthalene, toluene and cyclopentadiene, and aromatic hetereocyclic structures, such as quinoline, quinoxaline, isoquinoline, benzoquinoline, phenylquinoline, acridine, aminoacridine, phenazine, phenantridine, carbazole, benzimidazole, phenanthroline, phenoxazine, and acridone. Since the aromatic residue comprises at least two aromatic rings, it forms polycyclic planar structures, which have the property to intercalate into nucleic acid molecules due to their chemical structure. Intercalation occurs by insertion of the aromatic residue between adjacent base pairs of the nucleic acid molecule. Although intercalation does not disturb the stacking pattern of the nucleic acid, it nevertheless results in a vertical separation of the nucleic base pairs and in a partial unwinding of the nucleic acid molecule. Due to these conformational changes of the nucleic acid molecule, binding of other ligands, e.g. enzymes or transcription factors, is disturbed or prevented. The aromatic residues further can undergo non-intercalative stacking interactions with nucleobases of nucleic acids, given that these are accessible. Such interactions are often found at the termini of nucleic acid molecules and significantly increase the affinity of the complex.

Therefore, by introducing the ncAA of the invention into a peptide, the peptide obtains nucleic acid binding properties. Moreover, intrinsic DNA interacting capacities of proteins can be improved by incorporating the ncAA of the invention. Interestingly, the integration of rather small, non-charged chromophores into larger scaffolds, as e.g. peptides, promotes their nucleic acid binding activity, resulting in molecules with more balanced binding forces and higher selectivity compared to classical intercalators such as 4',6-diamidino-2-phenylindole (DAPI) or 3,8-diamino-5-ethyl-6-phenylphenanthridinium bromide (ethidium bromide).

In a preferred embodiment, the aromatic residue comprises at least three aromatic rings. Although intercalation of aromatic residues depends on various different chemical interactions, empiric guidelines for stacking and intercalation showed that the intercalation potential of aromatic residues increases with their size. Therefore, incorporating a ncAA with an aromatic residue having at least three aromatic rings provides a peptide of particular strong nucleic acid binding properties.

In a preferred embodiment, at least two aromatic rings of the aromatic residue are fused. This improves the intercalative potential of the residue, e.g. the probability of intercalation and the strength of the molecular interactions. Moreover, the integration of a rather small intercalating structure into a larger scaffold, as e.g. a peptide, improves the intercalative binding potential of the resulting molecule. Thus, the integration of a ncAA comprising two fused aromatic rings into a peptide provides a nucleic acid binding molecule with strong and reliable binding affinities. Accordingly, the aromatic residue is preferably selected from the group consisting of:

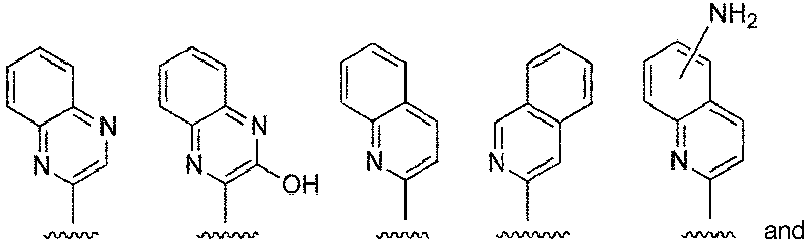

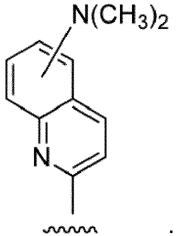

In a preferred embodiment, at least one of the aromatic rings comprises at least one heteroatom, preferably nitrogen. Aromatic residues comprising one, two or more nitrogen atoms were found to become particularly efficiently incorporated into peptides and proteins. It is believed that this is due to the chemical interactions of the heteroaromatic ring and the aminoacyl-tRNA synthetase within the ncAA binding pocket. In particular, ncAAs with chromophores comprising a single nitrogen atom but being otherwise unsubstituted were found to interact most efficiently with modified PylRSs. Moreover, due to the specific molecular interactions between the ncAA and the modified PylRS within the ncAA binding pocket, aromatic residues, which comprise a benzene ring with a nitrogen atom at position 4 and are fused to a further benzene ring at positions 5 and 6, are particularly preferred. Accordingly, the aromatic residue is preferably selected from the group consisting of:

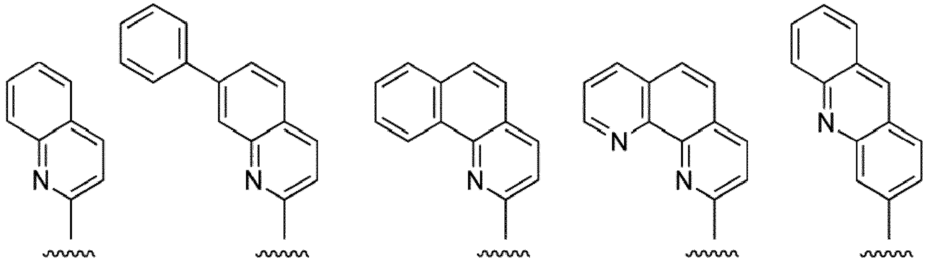

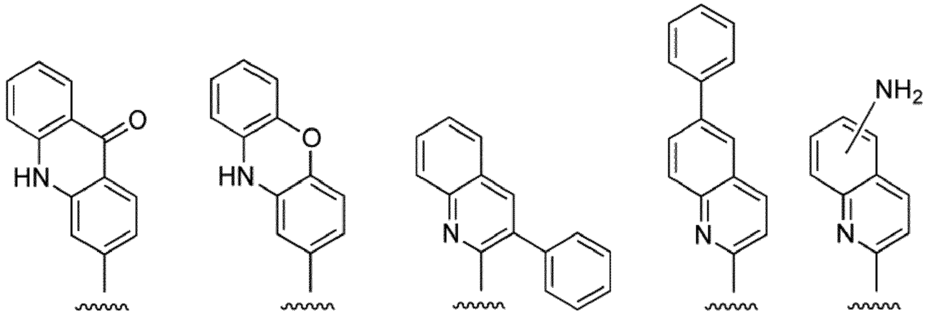

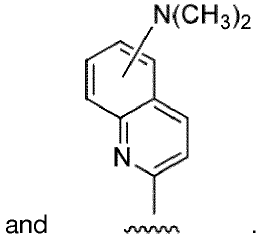

In a preferred embodiment, the aromatic residue is selected from the group consisting of quinoline, quinoxaline, isoquinoline, benzoquinoline, phenylquinoline, acridine, aminoacridine, phenazine, phenantridine, carbazole, and benzimidazole. These structures and their derivatives have polycyclic, charged and planar aromatic residues, which efficiently intercalate into nucleic acid structures. In particular, lysine derivatives comprising quinoline or quinoxaline residues were found to become reliably incorporated into proteins, e.g. GFP, by the use of a modified PylRS. Therefore, the aromatic residue is preferably selected from the group consisting of:

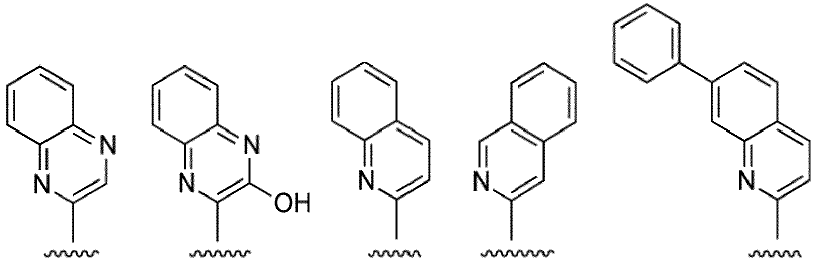

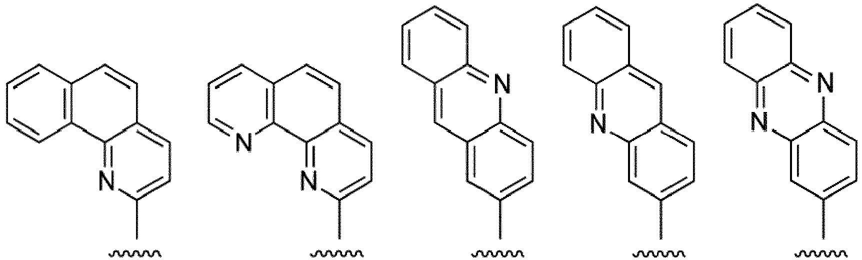

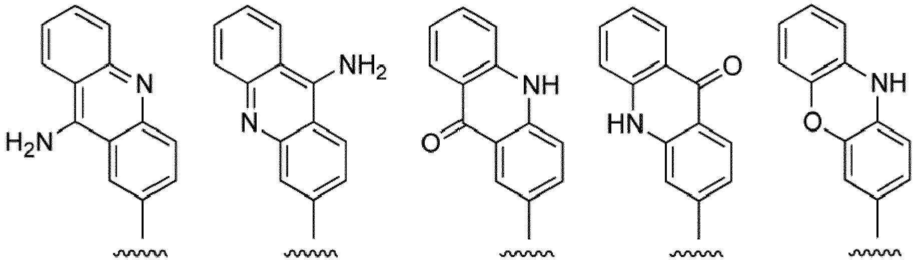

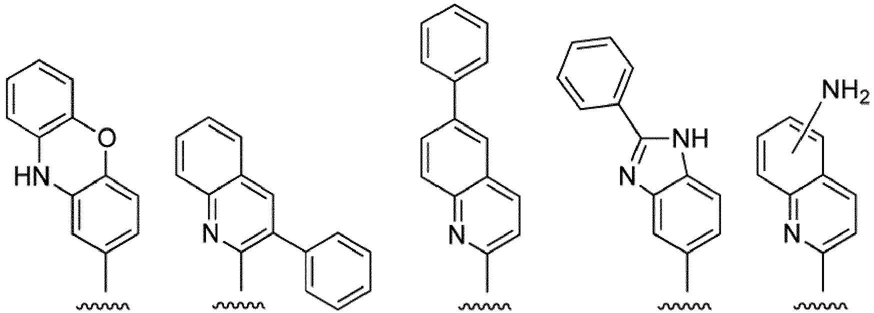

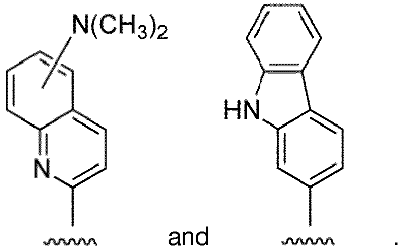

In a preferred embodiment, the aromatic residue is free of hydroxyl (-OH) substitutions. Aromatic residues, which do not have any -OH substitutions, as e.g. ncAA 6 (**Figure 1****)** were found to be more efficiently and accurately incorporated in peptides compared to -OH substituted equivalent.

In a preferred embodiment, the aromatic residue is bound to the N-epsilon atom of the lysine by an amide-, carbamate- or urea bond. Amine- and carbamate bonds are often found in ncAAs, and ncAAs comprising such structures are effective substrates for amino-acyl tRNA syntethases such as PylRS. Moreover, carbamate bonds can be used for introducing almost any aromatic residue, independently of the chemical structure of the residue. Accordingly, in a particularly preferred embodiment, the non canonical amino acid is selected from the group consisting of 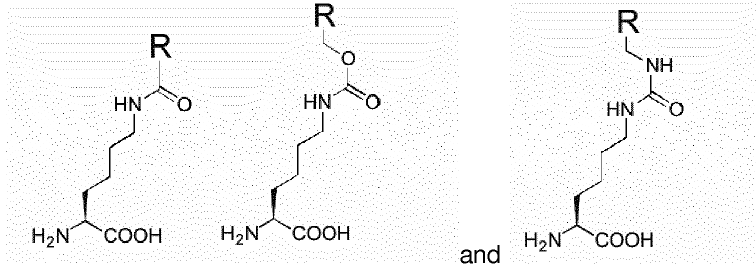 wherein R is the aromatic residue.

The ncAA may be produced by coupling a carboxylic acid derivative of the aromatic residue to a lysine. The carboxylic acid derivatives of the aromatic residues may be commercially purchased or produced by common methods. The coupling can be achieved, for example, by reacting the epsilon-N group of N alpha-tert-butoxycarbonyl-L-lysine-tert-butylester to the corresponding amide using benzotriazol-1-yl-oxy-tripyrrolidino-phosphonium-hexafluorophosphate (pyBOP). Subsequently, the α-amino and α-carboxyl groups of the resulting molecule are deprotected with trifluoroacetic acid.

In a further aspect, the invention relates to a ncAA selected from the group consisting of

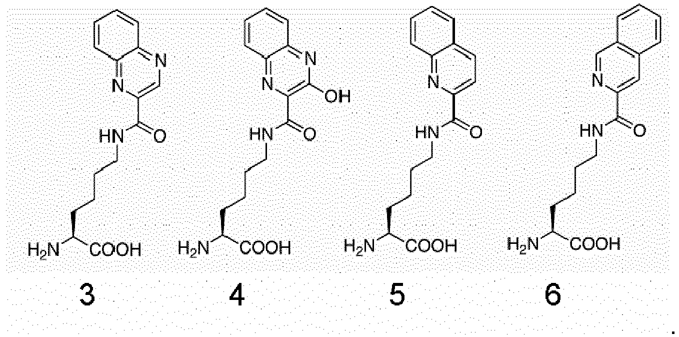

These lysine derivatives comprising quinoline or quinoxaline residues were found to become incorporated into peptides with high efficiency and accuracy. Of all investigated compounds, ncAA 6 showed the best results **(****Figure 2****).**

In a further aspect, the invention relates to a peptide comprising a ncAA of the invention. The ncAA is a derivative of a natural amino acid, namely lysine, and, thus, can be integrated into the amino acid sequence of any peptide. Moreover, since the amino acid resembles a natural amino acid and the aromatic residue is connected to the N-epsilon atom of the lysine, i.e. located in distance from the peptide's backbone, the ncAA is unlikely to interfere with the peptide's endogenous function. By integrating the ncAA into the amino acid sequence, any given peptide can be provided with the property to bind to nucleic acids. For example, the ncAA may be incorporated into GFP. The resulting GFP derivative binds to nucleic acids and is suitable for visualizing DNA and/or RNA. Likewise, ncAA comprising peptides are suitable for isolating nucleic acids, e.g. through antibodies specifically binding to the respective peptide. The peptide, upon mixing with a nucleic acid containing sample, binds to DNA and/or RNA via the aromatic residue. Subsequently, the mixture is brought into contact with a matrix coated with antibodies against the peptide, such that the peptides together with the bound nucleic acid molecules attach to the antibodies and are isolated from the sample. Additionally, the introduction of a ncAA into a peptide is suitable to direct the peptide to a specific DNA or RNA site. Several aromatic residues were found to intercalate into specific nucleic acid sequences. By integrating a ncAA comprising such a residue into a given peptide, the peptide can be specifically targeted to the respective DNA/RNA sequence.

In a preferred embodiment, the peptide is a nucleic acid binding drug, preferably for treating cancer, bacterial or viral infections. Besides the technological applications outlined above, the peptides of the invention are particularly suitable for medical uses. Many peptides are known to interfere with proteins having DNA or RNA binding affinities, such as proteins of the DNA replication system or transcription factors. Such peptides are often suitable for inhibiting cell proliferation and/or cell signaling and are therefore interesting drug candidates. However, those peptides having good inhibitory properties are often rather large molecules and therefore difficult to administer. Small peptides, in contrast, which can be introduced into a cell more efficiently, often show weak and unreliable DNA/RNA binding. By introducing the ncAA of the invention, however, the nucleic acid binding properties of such small peptides are significantly improved. This greatly increases their medical value. In addition, peptides, which interfere with nucleic acid binding molecules but do not show DNA/RNA binding themselves, can be provided with intercalating activity by the introduction of a ncAA of the invention. The peptides then also localize to the DNA and/or RNA, increasing the chance of efficient interference with the targeted DNA or RNA binding protein. For example, several peptides were found to have high binding affinity to topoisomerase 1 (top 1), but do not exhibit cytotoxic effects when used alone. However, upon administration together with the DNA intercalating 9-aminocamptothecin, the peptides cause great synergistic cytotoxic effects (Pond et al., 2006). By incorporating the ncAA of the invention, the top1 binding peptides can be provided with DNA binding activity, presumably inducing cytotoxic effects independently of 9-aminocamptothecin.

In summary, peptides comprising a ncAA of the invention provide potential drugs, in particular for inhibiting cell proliferation and inducing cell death. Accordingly, they are particularly suitable for treating cancer as well as bacterial and viral infections.

In a further preferred embodiment, the peptide comprises a RNA binding motif derived from a viral protein. A successful approach for the design of antiviral drug candidates is based on peptides and peptidomimetics derived from RNA binding motifs of essential viral proteins. Compared to common small molecules compounds, such peptides exactly match with the viral RNA. Thus, they generate a comparably large number of contacts with the viral RNA, such that they selectively bind the viral RNA and are able to disrupt large protein RNA complexes. A particular preferred target of the outlined strategy is the HIV-1 TAT/transactivation response (TAR) RNA complex (Davidson et al., 2009). However, the natural binding motifs of TAT as well as many other RNA binding viral proteins is particularly arginine rich, such that RNA binding mainly relies on non-directional electrostatic interactions. As a consequence, binding between the viral RNA and the peptide is rather unspecific. By incorporating a ncAA of the invention into a TAR targeting peptide, however, the binding selectivity and specificity of the peptide is significantly increased. For example, quinoline carboxamide-bearing fluorescently tagged TAT peptides were found to reliably recognize TAR RNA *in vitro* as indicated by electromobility shift assays **(****Figure 5****).**

In a preferred embodiment, the peptide is bound to a chemical substance, preferably selected from the group consisting of a labeling substance, preferably a fluorescent labeling substance or a radioactive labeling substance, a diagnostic substance, preferably a contrast agent, and a pharmaceutical substance, preferably for treating cancer, bacterial infections or viral infections. By binding the peptide to a chemical substance, the peptide is provided with further specific properties. Moreover, the peptide and the chemical substance can be specifically selected for individual applications.

For example, by binding the peptide to a labeling substance, the peptide and thus the bound nucleic acid can be detected, e.g. using a fluorometric device in case of a fluorescent labeling. Such peptides are particularly suitable to be used in fluorescence-based screening techniques.

Alternatively, the peptide may be used to deliver a diagnostic or pharmaceutical substance. For example, the diagnostic substance may be a contrast agent and the peptide may be specifically binding to the DNA of tumor cells. By incorporating the ncAA of the invention into the peptide, the binding affinity and specificity of the peptide is increased. After administration, the peptides accumulate in the tumor cells, where they can be detected by the contrast agent. Likewise, the specificity and strong DNA binding properties of the peptide may be used to specifically deliver drugs to their site of effect. Depending on the binding specificity of the peptide, such approaches may be employed for anti-cancer drugs as well as antibacterial and antiviral substances. Thus, binding a chemical substance to a peptide of the invention improves the efficiency and specificity of the substance's distribution in a body.

In a particular preferred embodiment, the chemical substance is a marker protein. The term "marker protein" as used herein refers to a protein that can be detected by its specific properties, e.g. by its fluorescence, its phosphorescence, its specific interactions with a reporter molecule, such as horseradish peroxidase, or by specific binding affinities as for example found for His-tag molecules. The peptide comprising a ncAA of the invention binds to DNA and/or RNA and can be detected through the marker protein. Such compounds provide useful tools for screening techniques, which commonly work with high throughput detection methods. Moreover, since both, the peptide and the marker protein, consist of amino acids, the entire compound can be expressed on block using cellular expression systems or cell-free translation assays. Importantly, no synthetic chemical reactions are needed to provide the compound with the different properties, i.e. the DNA binding affinity, which is mediated by the aromatic residue, and the detectability, which is mediated by the marker protein. In an even more preferred embodiment, the ncAA is directly incorporated into the marker protein.

In a preferred embodiment, the peptide is cyclized. Cyclization of peptide backbones results in increased proteolytic stability and membrane permeability of the peptide and, thus, improves medical administration. Moreover, cyclization further increases the binding affinity and specificity of the ncAA comprising peptide. Cyclization may be achieved by reacting the peptide with either α,α'-dibromo-m- or oxylene. This results in rapid and quantitative formation of stable non-polar thioether crosslinks, which improve bioavailability and impose differential steric constrains on the peptide backbone due to the use distinct xylene regioisomers.

In a further aspect, the invention relates to a method for enhancing the affinity of a nucleic acid binding peptide, comprising introducing a ncAA of the invention into the peptide, wherein the aromatic residue is a nucleic acid intercalating residue. By introducing a ncAA of the invention into a peptide, the peptide is provided with nucleic acid binding properties. Likewise, in the case a peptide already has, although weak DNA/RNA binding activity, this activity can be improved by incorporating a ncAA of the invention into the peptide.

In a further aspect, the invention relates to a method for producing a nucleic acid binding compound comprising the steps of providing a nucleic acid sequence encoding for a peptide, introducing a stop codon or a quadruplet codon into the sequence, expressing the sequence in the presence of a tRNA^{Pyl}, a modified PylRS, and a ncAA of the invention, which is encoded by the stop codon or the quadruplet codon, wherein the aromatic residue is a nucleic acid intercalating residue. Since the aromatic residue mediating the intercalation is fused to the lysine, it can be integrated into any peptide by common peptide bonds through the process of translation. This can be achieved either by using a cellular translation system or a cell-free translation system (also called *in vitro* translation system). The term "cellular translation system" as used herein refers to a cell or organism, which is modified such that it is suitable to express the peptide of the invention. The cell provides all molecules necessary for protein translation, in particular endogenous tRNAs, natural amino acids and ribosomes. Cells for protein production may be microorganisms, such as prokaryotic cells, preferably bacterial cells, or eukaryotic cells, preferably cells of a fungus. Cellular translation systems are commonly used for the production of recombinant proteins, with *Escherichia coli, Saccharomyces* and *Bacillus* most widely applied. The term "cell-free translation system" as used herein refers to *in vitro* systems comprising all compounds necessary for functional protein translation. The systems are commonly based on cell extracts, e.g. from *E. coli,* wheat germ or rabbit reticulocytes, and contain ribosomes, translation factors, and tRNAs. Alternatively, they can be reconstituted from individually expressed and purified components, as in the co-called PURE-system. Depending on the application, the systems may be supplemented with further compounds, e.g. RNA polymerases.

Using either translation system, a nucleic acid molecule encoding the peptide's sequence serves as a template (mRNA). For incorporating the ncAA of the invention into the peptide encoded by the mRNA, a unique codon is introduced into the mRNA at the position corresponding to the position of the ncAA in the peptide. This unique codon is assigned to the ncAA through the use of an orthogonal aaRS/tRNA pair. Thus, the genetic code is expanded by a further codon, which is translated by the protein translation machinery into the ncAA (Liu and Schultz, 2010). For ensuring specificity, the codon assigned to the ncAA is unique, preferably a stop codon or a quadruplet codon. In the course of translation, the ribosome produces an amino acid chain according to the nucleic acid sequence of the mRNA incorporating the ncAA.

When using cellular translation systems, the cells may be modified to express a tRNA responding to the unique codon and an aminoacyl-tRNA synthetase for transferring the ncAA to the tRNA. For example, the tRNA may be a tRNA^{Pyl}, which responds to an amber codon and the ncAA may be transferred thereon by a modified pyrrolysine-tRNA-synthetase (PyIRS), e.g. PylRS_AF. For cell-free translation assays, the assay may be supplemented with ncAA already loaded onto a tRNA.

In a preferred embodiment, the method further comprises the step of linking the peptide to a chemical substance. This allows the adaption of the peptide for specific applications as outlined herein.

In a preferred embodiment, the sequence is expressed in a cellular translation system or an *in vitro* translation assay, preferably using a modified PylRS. When using a cellular translation system, such as a microorganism, it is sufficient to provide the cells with nucleic acid sequences encoding the tRNA, the aminoacyl-tRNA synthetase and the mRNA of the protein, and supplement the medium of the cells with the ncAA. The ncAA is transported into the cells' cytoplasm where it is transferred onto the tRNA by the aminoacyl-tRNA synthetase. Moreover, most microorganisms are easy to culture in large quantities and techniques for harvesting the cells and isolating the peptide are available.

In cell-free translation systems, in contrast, all components need to be added as functional molecules or their subunits. However, the translation process can be controlled more stringently in such systems and isolation of the produced peptides is easier.

The term "modified PylRS" as used herein refers to a pyrrolysine-tRNA-synthetase, which differs from the wild type PylRS (SEQ ID NO.: 3) by amino acid exchanges that result in an enlargement of the ncAA binding pocket. This may be achieved, for example, by replacing tyrosine at position 306 by alanine or glycine, which are both much smaller amino acids. Such an exchange opens a pocket along the alpha6 helix up to the η13₁₀-helix that is shielded by the tyrosine (Y306) in the wild type PylRS. As a consequence and unlike found for pyrrolysine, a ncAA according to the invention, as for example ncAA 6, fits into the binding pocket of the PylRS. In case of ncAA, it was found that, in contrast to pyrrolysine in wild type PylRS, the alpha carboxyl of ncAA 6 is not positioned for inline nucleophilic attack of the alpha phosphate but in a very large distance of 5.1 Å. This results in a shift of the complete ncAA 6 into the rear part of the pocket by several Å that leads to a displacement of the N-epsilon carbonyl group by 4 Å relative to the position found for pyrrolysine. Additionally, the pyridyl ring of the quinoline residue is not in a position similar to the methylpyrroline ring of the pyrrolysine but placed behind it.

In a further aspect, the invention relates to a kit comprising a ncAA of the invention, a nucleic acid encoding tRNA^{Pyl}, and a nucleic acid encoding a modified PylRS. The kit provides the components needed for producing a peptide of the invention, using a cellular translation system. For producing the protein in a cellular translation system, the nucleic acids of the kit are introduced into the cells of the translation system, e.g. by vector transformation or stable integration into the DNA of the cells. In addition, the culture media of the cells is supplemented with ncAAs supplied with the kit. The ncAAs are transferred into the cells' cytoplasm, where they are processed by the modified PylRS and loaded onto the tRNA^{Pyl}. The resulting loaded tRNA is then used by the endogenous ribosome for protein production. Since the tRNA^{Pyl} corresponds to a stop or quadruplet codon, the ncAA will only be introduced into the given peptide. The kit is also suitable for producing the peptide in a cell-free translation assay, provided that the assay is competent to produce tRNA and PylRS from encoding nucleic acids.

In a further aspect, the invention relates to the use of a ncAA comprising a lysine and an aromatic residue for producing a peptide, wherein the aromatic residue is bound to the N-epsilon atom of the lysine and comprises at least two aromatic rings.

In a further aspect, the invention relates to a modified pyrrolysyl-tRNA-synthetase (PylRS) having a substitution of alanine or glycine for tyrosine at position 306, wherein the PylRS further comprises a substitution of alanine or glycine for lysine at position 407 and/or a substitution of alanine or glycine for aspartic acid at position 408. In addition to PylRS_AF, the inventors developed further alternative modified PylRSs which are suitable to transfer ncAAs comprising an aromatic residue with at least two aromatic rings on a tRNA^{Pyl}. These modified PylRS harbor a Y306A or Y306G mutation and an additional amino acid substitution at position 407 and/or 408 (PylRS_AF_L407A: SEQ ID NO.: 5; PylRS_AF_ D408A: SEQ ID NO.: 6). As a result of these substitutions, the ncAA binding pocket is even further enlarged compared to PylRS_AF, accompanied by a reduced steric demand, such that the binding pocket is suitable to accept lysine derivatives with even larger aromatic residues, e.g. comprising at least three aromatic rings.

In a further aspect, the invention relates to a nucleic acid encoding for a modified PylRS of the invention, to a vector comprising such a nucleic acid and to a microorganism comprising such a nucleic acid. The modified PylRS may be produced using cell-based systems or cell-free assay.

### Examples

### Experimental procedures

Materials and Methods for Chemical Synthesis. All reactions were performed under anhydrous conditions and an inert atmosphere of argon in oven-dried glassware with magnetic stirring. Yields refer to chromatographically and spectroscopically (1H NMR) homogenous materials. Reagents were used as obtained from typical commercial sources. Flash chromatography was carried out using Roth Kieselgel 60 F254 (230-400 mesh) silica gel. All reaction solvents were purchased in anhydrous quality and stored over molecular sieve. 1 H and 13C NMR spectra were recorded at ambient temperature on a Bruker Avance III 400 instrument. Signal positions were reported in δ/ppm. High-resolution mass spectrometry (HRMS) measurements were performed on a Bruker Daltonics micrOTOF II. General Synthesis Procedure for Nε-modified Nα-tertbutoxycarbonyl-L-lysine-tert-butylesters 3a - 8a. Nα-tertbutoxycarbonyl-L-lysine-tert-butylester (0.5 g, 1.65 mmol) and the carboxylic acid of the respective chromophore (1.65 mmol, 1 eq) were placed in a 50 ml two-necked round bottom flask and were dissolved in anhydrous dichloromethane (25 ml) under argon atmosphere. The solution was cooled to 0 °C in an ice bath and N-methyl-morpholine (0.4 ml, 3.64 mmol, 2.2 eq) and PyBOP (0.95 g, 1.82 mmol, 1.1 eq) were added. The reaction mixture was stirred for 16 h at room temperature. The solution was washed with 30 ml NaH-CO₃, the phases were separated and dried with MgSO₄. The solvent was removed under reduced pressure and the obtained oil was purified using flash column chromatography with ethyl acetate / petrol ether mixtures. The pure product was obtained as a foam. General Synthesis Procedure for Nε-modified L-lysine derivatives ncAA 3 - 8. (1.33 mmol) were dissolved in 8 ml TFA and the mixture was stirred 3 h at room temperature. TFA was removed and the residue was coevaporated once with methanol. The residue was dissolved in a minimal amount of methanol and precipitated with cold diethyl ether. The precipitate was filtered, washed with diethyl ether and dried under reduced pressure. ncAA 3 - 8 were obtained as the TFA salt.

Plasmid Constructions. For construction of pREP_PylRS_wt, pREP_PylRS_GF and pREP_PylRS_AF, a Pstl site and a Sphl site were removed from plasmid pREPCM3+RBS64 by Quickchange site directed 7 using primers UPRT-Pstl_fwd and UPRT-Pstl_rev and primers Tet_Sphl_fwd and Tet_Sphl_rev, resulting in pDaS80. A cassette containing *M*. *mazei* tRNA^{Pyl} under control of a proK promoter and *M*. *mazei* PylRS_wt _GF or _AF under control of the glnRS'-promoter was amplified from pEVOL_PylRS_wt and pEVOL_PylRS_AF using primers Xbal_pEVOL-5'_PCR_primer and Sphl_pEVOL-3'-PCR_primer and cloned into the Xbal/Sphl sites of pDaS80. An EcoRI site was removed from the obtained plasmids using primers PylRS_Quickch_EcoRI_fwd and PylRS_Quickch_EcoRI_rev. This resulted in plasmids pREP_PylRS_wt and pREP_PylRS_AF. pREP_PylRS_GF was constructed by introducing the Y306G mutation into pEVOL_PylRS_wt using primers PylRS_Y306G_fwd and PylRS_Y306G_rev. pREP_PylRS_AF_L407A and pREP_PylRS_AF_D408A were obtained by Quickchange mutagenesis using primers PylRS_L407A_fwd and PylRS_L407A_rev or PylRS_D408A_fwd and PylRS_D408A_rev, respectively. For construction of pBAD_TRX_wt and pBAD_TRX_R74TAG, the *E*. *coli* thioredoxin gene with an N-terminal region coding for an S-tag and downstream enteropeptidase cleavage site was amplified from plasmid pET32a-TRXtag (Addgene) with primers Ncol-TRX_fwd and Notl-TRX-rev and cloned into the Ncol and NotI sites of plasmid pBAD-GFP39TAG26, resulting in plasmid pBAD-TRX-His6. An amber mutation at site R74 was introduced by Quickchange site directed mutagenesis using primers TRX-R74TAG_fwd and TRXR74TAG_rev, resulting in plasmid pBAD-TRX-His6_R74TAG. For construction of plasmid pBAD_CmodC, a synthetic peptide gene was obtained by primer extension using template Ncol_Mod_Nhel_fwd and primer Ncol_Mod_Nhel_rev and ligated into the same sites in pBAD_GFP-wt-NheI+, replacing the N-terminal flag tag. For construction of pPylRS_AF_188-454-His6, a C-terminal fragment of *M*. *mazei* PylRS_AF consisting of amino acids 188 to 454 with N-terminal His6-tag was amplified from pE-VOL-PylRS_AF using primers BglII_PylRS_short_fwd and SalI_PylRS_rev. This fragment was cloned into the Bglll and SalI sites of pEVOL-PylRS_AF, resulting in plasmid pPylRS_AF-188-454-His6. For construction of pBAD_TAT, a cloning fragment was obtained by primer extension using template TAT_Natmin_TAG6 and primer TAT_rev and cloned into the Ncol and Nhel sites of pBAD_GFP-wt-Nhel+.

### Primer sequences:

| Primer | Sequence | SEQ ID NO. |
|---|---|---|
| UPRT-PstI_fwd | ATC GAC CTG CTG AAA AAA GCG GGC TGT AGC AGC ATC AAA G | 7 |
| UPRT-PstI_rev | GCA CCA GAA CTT TGA TGC TGC TAC AGC CCG CTT TTT TCA GC | 8 |
| Tet_Sphl_fwd | ATC TCC TTA CAT GCA CCA TTC CTT GCG GCG GCG | 9 |
| Tet_Sphl_rev | GCA AGG AAT GGT ACA TGC AAG GAG ATG GCG CCC AAC | 10 |
| Xbal_pEVOL_5I-PCR_primer | TTT TTT CTA GAA TCA ATC ATC CCC ATA ATC CTT GTT | 11 |
| Sphl_pEVOL-3I-PCR_primer | TTT TTG CAT GCA GGC AGT TAT TGG TGC CCT TAA AC) | 12 |
| PylRS_Quickch_EcoRI _fwd | CGA AAT CAG CCT GAA CTC CGG CAA ACC GTT TCG TGA ACT | 13 |
| PylRS_Quickch_EcoRI _rev | AAA CGG TTT GCC GGA GTT CAG GCT GAT TTC GTC TTT CGG A | 14 |
| PylRS Y306G_fwd | CGC CCT ATG CTA GCA CCA AAT CTG GGT AAC TAT CTG CGC AAA C | 15 |
| PylRS Y306G_rev | GCA CGG TCC AGT TTG CGC AGA TAG TTA CCC AGA TTT GGT GCT A | 16 |
| Ncol-TRX_fwd | TTT TCC ATG GGC GAT AAA ATT ATT CAC CTG ACT | 17 |
| Notl-TRX-rev | TTT TGC GGC CGC TCA ATG ATG ATG ATG ATG ATG CTT GTC | 18 |
| TRX-R74TAG_fwd | CGA AAT ATG GCA TCT AGG GTA TCC CGA CTC TGC TGC TGT TCA AA | 19 |
| TRX-R74TAG_rev | CAG CAG AGT CGG GAT ACC CTA GAT GCC ATA TTT CGG CGC A | 20 |
| Ncol_Mod_Nhel_fwd | TTT CCA TGG GCT GTC TGC GTT TTG GCT AGA GCT GCG GCG GTA GCG CTA GCT TT | 21 |
| Ncol_Mod_Nhel_rev | AAA GCT AGC GCT ACC GCC | 22 |
| BglII_PylRS_short_fwd | TTT TTA GAT CTA TGC CAG CAC TGA CAA AAT CCC AAA CC | 23 |
| SalI_PylRS_rev | TTT TTG TCG ACT CAA TGA TGA TGA TGA | 24 |
| | TGA TGC AGG TTC GTA GAG ATC | |
| PylRS_L407A_fwd | GTT GGA CCA ATT CCG GCC GAC CGT GAG TGG GGT ATC | 25 |
| PylRS_L407A_rev | CCC CAC TCA CGG TCG GCC GGA ATT GGT CCA ACA ACG GC | 26 |
| PylRS_D408A_fwd | GGA CCA ATT CCG CTG GCC CGT GAG TGG GGT ATC GAC | 27 |
| PylRS_D408A_rev | CCC CAC TCA CGG GCC AGC GGA ATT GGT CCA ACA ACG | 28 |
| TAT_Natmin_Cys_TAG 6 | CGA TTT TTC CAT GGG CTG CTA TGG CTA GAA AAA ACG CCG CCA GCG CCG CCG CTG CGG CGG CTC GGC TAG CTT TTT | 29 |
| TAT_rev | AAA AAG CTA GCC GAG CC | 30 |

Protein Expression and Purification. All proteins were expressed from pBAD vectors and purified using a C-terminal His6 tag. For expression of proteins containing ncAA, *E*. *coli* cells were co-transformed with pBAD plasmid containing the target gene with amber codon (pBAD_GFP-39TAG, pBAD_TRX-R74-His6_TAG, pBAD_CmodC or pBAD_TAT, all conferring ampicillin resistance) and a plasmid coding for the respective PylRS mutant and tRNA^{Pyl} (pEVOL_PylRS_AF, pREP_PylRS_wt, _GF, _AF and PylRS_AF single or double alanine mutants). A single clone was picked and grown in Luria Broth (LB) media supplemented with suited antibiotics at 37 °C and 200 rpm shaking. 0.1 ml of this culture was used to inoculate 5 ml LB supplemented with the same antibiotics and different concentrations of the ncAA. The culture was incubated at 37 °C and 200 rpm shaking until OD600 of ∼0.4 - 0.6 and protein expression was induced with 0.02% (w/v) L-arabinose. The culture was harvested after 4 - 6 h of incubation by centrifugation (10 min, 3320 x g, room temperature). The pellet was lysed with B-Per lysis reagent (Thermo scientific) containing 1x Complete (-EDTA) protease inhibitor cocktail (Roche) and shaking at room temperature at 1400 rpm for 30 min. The suspension was pelleted by centrifugation (5 min, 20817 x g, room temperature) the supernatant was extracted with Ni-NTA agarose slurry (Qiagen) at an imidazole concentration of 10 mM by shaking at room temperature. The beads were washed with 350 µl 4x PBS and or two wash buffers (50 mM NaH₂PO₄, 300 mM NaCl, pH = 8.0, containing 20 - 50 mM imidazole). Proteins were eluted with elution buffer (50 mM NaH₂PO₄, 300 mM NaCl, pH = 8.0, containing 500 mM imidazole). For GFP-TAT protein containing ncAA 6, expressions were carried out in BL21 (DE3) gold and purification was conducted as above, but under denaturing conditions (8 M urea). Protein was refolded by stepwise dialysis against 20 mM Tris-HCl, pH = 8.0, 10 % glycerole with decreasing urea concentrations. Purity of the obtained proteins was analyzed by SDS PAGE and staining with Gelcode blue (Thermo Scientific) and quantified using a BCA assay (Pierce, Thermo Scientific). For GFP yield determination based on whole cell fluorescence, cell pellets from 3 ml expressions were washed twice with 3 ml 4x PBS (by centrifugation for 10 min at 3320 x g at room temperature and resuspension) and fluorescence was determined using a Tecan M200 plate reader using and excitation wavelength of 475 nm and an emission wavelength of 510 nm. Fluorescence values were corrected for cell densities based on OD600 - measurements. Generally, expressions were carried out in *E. coli* Top10 using carbenicillin selection for pEVOL plasmids and tetracyclin selection for pREP plasmids. Wild type thioredoxin and TRX-R74→6 were expressed in *E. coli* BL21 (DE3) gold cells. Expressed C-terminal fragment of PylRS_AF (aa 188-454) was stored in 10 mM HEPES-Na+, pH = 7.5, 5 mM MgCl2, 300 mM NaCl at -80 °C before use.

Protein Mass Spectrometry. All protein samples were dialyzed against water using Slide-A-Lyzer mini dialysis units (Pierce, Thermo Scientific) with a molecular weight cut-off of 10.000 Da before measurement. Tryptic protein digests were analyzed by reversed phase liquid chromatography nanospray tandem mass spectrometry (LC-MS/MS) using an LTQ-Orbitrap mass spectrometer (Thermo Fisher) and an Eksigent nano-HPLC. The dimensions of the reversed-phase LC column were 5 µm, 200 Å pore size C18 resin in a 75 µm i.d. x 10 cm long piece of fused silica capillary (Hypersil Gold C18, New Objective). After sample injection, the column was washed for 5 min with 95% mobile phase A (0.1% formic acid) and 5 % mobile phase B (0.1% formic acid in acetonitrile), and peptides were eluted using a linear gradient of 5 % mobile phase B to 40% mobile phase B in 65 min, then to 80% B in an additional 5 min, at 300 nL/min. The LTQ-Orbitrap mass spectrometer was operated in a data dependent mode in which each full MS scan (30 000 resolving power) was followed by five MS/MS scans where the five most abundant molecular ions were dynamically selected and fragmented by collision-induced dissociation (CID) using a normalized collision energy of 35% in the LTQ ion trap. Dynamic exclusion was allowed. Tandem mass spectra were searched against a homemade protein database using Mascot (Matrix Science) with "Trypsin/P" enzyme cleavage, static cysteine alkylation by iodoacetamide and variable methionine oxidation. For measurements of whole proteins the MS/MS fragmentation was switched off and the resolution of the Orbitrap analyser was set to 7500. Deconvolution of protein spectra was performed using ProMass Deconvolution V2.5 software. Matrix-assisted laser desorption/ionization time of flight (MALDI-TOF) MS measurements were carried out with a Bruker Microflex MALDI-TOF spectrometer using a-cyano-4-hydroxycinnamic acid as matrix.

Generation of Cyclized Peptides. The Peptide-GFP fusion protein obtained from expressions using *E. coli* Top10 cotransformed with plasmids pBAD_CmodC and pEVOL_PylRS_AF was dialyzed against TEVHis6 reaction buffer (50 mM Tris-HCl pH=8, 0.5 mM EDTA, 1 mM DTT) overnight. 50 µl of this protein were digested with 1 µl (10 U) TEVHis6 protease (Invitrogen, Life technology) for 2.5 h at 30 °C. The reaction mixture was added to 50 µl Ni-NTA agarose slurry (Qiagen) in a paper filter spin column (Pierce) and incubated for 20 min at room temperature at 500 rpm. The cleaved peptide containing the ncAA was isolated by centrifugation of the resin (1 min, 20817 x g, room temperature). For cyclization, 25 µl of the peptide was added to 25 µl of a reaction mixture containing final concentrations of 20 mM (NH₄)₂CO₃ pH = 8.6, 200 µM (tris(2-carboxyethyl)phosphine), 1.1 mM α,α'-dibromo-o-xylene or α,α'-dibromo-m-xylene (Acros) and 25% acetonitrile (Sigma). The cyclization reaction was incubated for 1 h at 30 °C.

Electromobility Shift Assays. Synthetic TAR RNA was purchased (IBA,) and purified by 12% denaturing PAGE (8M urea). RNA was 5'-end labeled using γ-32P-ATP and T4 polynucleotide kinase and purified using a G-25 gel filtration column (GE healthcare). For binding, 10 nM RNA was incubated with varying concentrations of GFP-TAT protein containing ncAA 6 in 50 mM Tris-HCl pH = 7.5, 100 mM NaCl, 1 mM DTT and 5 % glycerole at 4 °C for 20 min, loaded onto an analytical, non denaturing PAGE gel and was run in 1x TB at 5 W and 4 °C. Gel was dried and data recorded on a phosphorimager.

PylRS Crystallization and X-Ray Crystallographic Data Acquisition and Processing. Crystals of PylRS_AF complexed with adenosine-5'-(β,γ-imido)triphosphate (AMPPNP) were grown by the vapor-diffusion hanging drop method at 18°C. Purified PylRS_AF was concentrated to 10 mg/ml, supplemented with 10 mM AMPPNP and mixed in a 1:2 ratio with reservoir solution (100 mM Tris pH 7.5, and 10% (w/v) PEG 2000 monomethyl ether). Crystals appeared overnight and grew to a maximum size of 150x100x50 µm. Cryostabilization of the crystals was done by soaking in reservoir solution supplemented with 2% additional PEG2000MME, 5 mM MgCl₂, 10 mM AMPPNP and increasing concentrations of ethylene glycol (final concentration 30% (v/v)). Cryostabilized crystals were flash frozen in liquid nitrogen and diffraction data were measured at the Swiss Light Source, Villigen, Switzerland. The crystals diffracted to 1.6 Å resolution. XDS was used to process diffraction data. The protein crystallized in space group C121 with one molecule in the asymmetric unit (unit cell dimensions: a=101.869Å, b=44.33Å, c=63.879Å and α=90°, β=100.44°, γ=90). The structure was solved by molecular replacement with PHASER using PDB entry 2Q7E) as search model. Several rounds of building and refinement were carried out with COOT and PHENIX, respectively.

### Synthesis and Genetic Encoding of ncAA Bearing Quinoline- and Quinoxaline-Derived Two-Ring Chromophores.

Because of its essential role for DNA-binding of the archetype DNA bisintercalator echinomycin²⁹, the inventors first aimed at the synthesis and genetic encoding of an ncAA bearing a quinoxaline-2-carboxamide chromophore **(****Figure 2****,** ncAA 3). For synthesis of ncAA 3, quinoxaline-2-carboxylic acid was coupled to lysine by reacting the ε-amino group of Nα-tert-butoxycarbonyl-L-lysine-tert-butylester to the corresponding amide using benzotriazol-1-yl-oxy-tripyrrolidinophosphonium-hexafluorophosphate (PyBOP). Subsequent deprotection of the α-amino and α-carboxyl groups with trifluoroacetic acid yielded Nε-(quinoxaline-2-carbonyl)-L-lysine 3 in 75 % overall yield. ncAA 4 - 7 were synthesized analogously from commercially available carboxylic acids.

Next, the tRNA^{Pyl}/PylRS pair of *Methanosarcina mazei* was analyzed for its capability to mediate the selective incorporation of ncAA 3 into proteins in *E*. *coli* in response to the amber codon, TAG. *E*. *coli* were co-transformed with plasmids pEVOL-PylRS_wt (encoding wild type M. *mazei* PylRS and tRNA^{Pyl}) and pBAD_GFP-39TAG (encoding GFP with an amber mutation at position Y39 and a C-terminal His6 tag) and the culture induced with L-arabinose in the presence or absence of 1 mM ncAA 3 or Nε-(tert-butyloxycarbonyl)-L-lysine (Boc-Lys, ncAA 2), which is an efficiently processed substrate of PylRS_wt. However, expression of full-length GFP was only detected for ncAA 2 by cellular fluorescence. A likely reason for this failure of amber suppression is poor accommodation of the large and rigid side chain of ncAA 3 within the binding pocket of PylRS_wt. Among amino acids that constitute the methyl-pyrroline binding pocket of PylRS, Y306 is a main component of the rear part and plays a critical role in discriminating against larger, Nε-carbamate-linked lysine derivatives. The inventors thus tested a PylRS mutant with reduced steric demand at this position (Y306A) combined with a Y384F mutation (PylRS_AF) for activity towards ncAA 2and 3 as described above. In fact, GFP-Y39→3 was now expressed at a yield of 1.4 mg/L **(****Figure 2****).** To test, if the presence of a hydroxy group as found in the second archetype DNA-bisintercalator sandramycine would be tolerated, the investors tested the respective derivative of ncAA 3 (Nε-(2-hydroxyquinoxaline-3-carbonyl)-L-lysine, **Figure 1****).** However, no significant fluorescence increase compared to background was detectable, indicating that the presence of a 3-hydroxyl group is unfavourable. Next, the role of the number and position of the aromatic nitrogen atoms was analyzed by testing Nε-(quinoline-2-carbonyl)-L-lysine (ncAA 5) and Nε-(isoquinoline-3-carbonyl)-L-lysine (ncAA 6). GFP-Y39→6 was expressed with a yield comparable to ncAA 3 (1.4 mg/L). However, quinolinecarboxamide ncAA 5 proved to be a highly efficient substrate and afforded 4.5 mg/L of purified GFP-Y39→5 under the employed non-optimized conditions (>11 mg/L were obtained under standard expression conditions). This suggested that a single nitrogen atom in an otherwise non-substituted chromophore is ideal and that its relative position to the fused benzene ring is important. Finally, to analyze the role of the nitrogen atom in ncAA 5 and ncAA 6, the inventors tested Nε-(naphtalene-2-carbonyl)-L-lysine (ncAA 7). However, this ncAA exhibited limited solubility (∼100 µM) and thus, expectedly, no expression of GFP-Y39→7 was observed **(****Figure 2****)**

Further, the inventors analyzed the role of the binding pocket properties of PylRS at position 306 for efficiency and fidelity of amber suppression with ncAA 5. The inventors performed expressions using *E. coli* co-transformed with pBAD_GFP-Y39TAG and pREP-PylRS_wt, AF and GF, coding for *M*. *mazei* tRNA^{Pyl} and the respective PylRS, where PylRS_GF potentially offers further reduced steric demand by a Y306G instead of Y306A mutation. SDS PAGE analysis of Ni-NTA-purified GFP extractions obtained from cultures expressing PylRS_wt indicated that ncAA 5, as previously ncAA 3, was not recognized **(****Figure 3 A)****.** In contrast, significant expression was observed for ncAA 5 and ncAA 2 with both PylRS_AF and PylRS_GF. With PylRS_GF, ncAA 5 afforded higher levels of GFP than ncAA 2. However, expression was generally higher for both ncAAs with PylRS_AF and showed a similar ratio for GFP-Y39→5 versus GFP-Y39→2 expression as observed in cellular fluorescence measurements. No expression could be detected in absence of ncAA 5, indicating high incorporation fidelity. Electrospray ionization tandem mass spectrometry (ESI MS/MS) of a purified, trypsin-digested GFP-Y39→5 sample from an expression with PylRS_AF confirmed the identity and site-selective incorporation of ncAA 5 at position Y39 by the found peptide mass (1623.72844 Da, theoretical mass = 1623.72845) and expected fragment ions (identity and site selective incorporation was confirmed in the same way for ncAA 3 and 7). To further assess the scope and fidelity of PylRS_AF-mediated incorporation of ncAA 5 into proteins, protein expression was performed using *E*. *coli* cotransformed with plasmids pEVOL_PylRS_AF and pBAD_TRX-His6-R74TAG (coding for *E*. *coli* thioredoxin (TRX) with an amber site at position R74 and a C-terminal His6-tag) in presence or absence of ncAA 5. Ni-NTA purification and SDS PAGE indicated high fidelity of incorporation **(****Figure 3 C)****.** ESI MS analysis of the purified protein confirmed the identity of ncAA 5 and again indicated high incorporation fidelity **(****Figure 3** **C;** mass difference of 127 Da between TRX wt and TRXR74→5 corresponds to mass difference between R and ncAA 5).

### Incorporation of a Quinolinecarboxamide Chromophore into Cyclized Peptides.

In view of the design of nucleic acid targeting drug candidates, the inventors aimed at incorporating ncAA 6 into cyclized peptides in a way that is compatible with evolutionary design strategies. Cyclization of peptide backbones often results in improved proteolytic stability, binding affinity/selectivity and membrane permeability. *E*. *coli* was co-transformed with plasmids pEVOL_PylRS_AF and pBAD_CmodC (encoding GFP fused to an N-terminal peptide that contains a single amber site and two cysteines) in presence of ncAA 5 and cleaved the purified construct with tobacco etch virus protease (TEV-His6, **Figure 4 A)****.** For cyclization, the peptide was reacted with either α,α'-dibromo-m- or o-xylene. This is expected to result in the rapid and quantitative formation of stable, nonpolar thioether crosslinks that may improve bioavailability and impose differential steric constraints on the peptide backbone due to the used distinct xylene regioisomers. Additionally, α,α'-dibromo-m-xylene and related reagents for cysteine crosslinking have been successfully employed in the evolutionary design of cyclized peptides by phage- and mRNA-display, which both are also well established for use with ncAA. Analysis by matrix-assisted laser desorption/ionization time of flight (MALDI-TOF) MS indeed showed the disappearance of the signal corresponding to the linear peptide precursor (2320.1 Da) and the occurrence of a new signal with a mass corresponding to the m- or o-xylene-cross-linked peptides, respectively (2424.2 and 2424.3 Da) (**Figure 4** **B**). This indicates high efficiencies of crosslink formations and establishes a simple route to the design of cyclized, quinolinecarboxamide-bearing peptides that is compatible with strategies of encoded, evolutionary design.

### Incorporation of a Quinolinecarboxamide Chromophore into the Arginine-Rich Motif of HIV-1 TAT.

A recent successful approach for the design of antiviral drug candidates relies on peptides and peptidomimetics derived from the RNA-binding motifs of essential viral proteins (Davidson et al., 2009). Compared to small molecules, these inhibitors provide a larger number of contacts, associated with the potential for increased selectivity and ability to disrupt protein-RNA complexes with large binding surfaces. Owing to its high conservation among viral isolates and its ubiquitous, vital role in viral transcription, the HIV-1 TAT/transactivation response (TAR) RNA complex has been a central target of that strategy. However, the natural binding motifs of TAT and many other RNA-binding viral proteins are arginine-rich and use nondirectional electrostatic interactions as main binding forces, which represent a potential source of nonspecific binding. The inventors aimed to expand the nucleic acid recognition repertoire of TAR-binding peptides by incorporating quinolinecarboxamide chromophores in a way that is compatible with highly parallel screening approaches. *E. coli* cells were co-transformed with plasmids pEVOL_PylRS_AF and pBAD_GFP-TAT (encoding a minimal motif of HIV-1 TAT (YGXKKRRQRRR, SEQ ID NO.: 4) containing a single amber codon (X) fused to a C-terminal GFP gene, (**Figure 5** **A**) in presence of ncAA 5. The obtained quinolinecarboxamide-bearing, fluorescently tagged TAT peptide was able to recognize TAR RNA *in vitro* as indicated by electromobility shift assays (EMSA) (**Figure 5** **B**). This indicates that the GFP tag does not significantly interfere with TAR binding, which provides a starting point for the creation of large diversities of arginine-rich peptides with expanded RNA-recognition potential that can be subjected to highly parallel fluorescent screening techniques.

### A PylRS Mutant with a New, Strongly Enlarged Binding Pocket.

To gain insights into the differential recognition of ncAA 3 - 7 by PylRS_AF and to establish a basis for further engineering efforts aiming at the genetic encoding of extended three-ring chromophores, the crystal structure of a C-terminal fragment (amino acids 188 - 454) of PylRS_AF in complex with adenosine-5'-(β,γ-imido)triphosphate (AMPPNP) was solved at 1.6 Å resolution (Table 1).

**Table 1: Data collection and refinement characteristics for crystal structure of PylRS_AF in complex with AMPPNP. Values in parentheses represent data from the highest resolution shell.**

| | |
|---|---|
| Wavelength (Å) | 1.0000 |
| Resolution range (Å) | 36.11 - 1.6 (1.55 - 1.497) |
| Space group | C 1 2 1 |
| Unit cell dimensions | |
| *α*, *b*, and *c* (Å) | 101.869 44.33 63.879 |
| *α*, *ß*, and *γ* (°) | 90100.4490 |
| Total reflections | 145757 |
| Unique reflections | 44440 (3930) |
| Completeness (%) | 97.86 (87.26) |
| Mean I/sigma(I) | 11.06 (1.34) |
| Wilson B-factor | 18.27 |
| R-work | 0.1884 (0.4878) |
| R-free | 0.2205 (0.5030) |
| Numberofatoms | 4365 |
| Protein residues | 259 |
| RMS (bonds) | 0.011 |
| RMS (angles) | 1.31 |
| Ramachandran favored (%) | 99.6 |
| Ramachandran outliers (%) | 0.4 |
| Average B-factor | 36.3 |
| macromolecules | 35.6 |
| solvent | 46.6 |

The overall structure showed less than 0.6 Å root mean square (rms) deviation against a reported structure of *M. mazei* wt PylRS in complex with AMPPNP (pdb-entry 2Q7E). The conformations of the flexible loops are highly conserved, in particular the β7-β8 loop exhibits a very similar conformation despite the contained Y384F mutation. Additionally, the conformations of AMPPNP and amino acid side chains forming the pyrroline ring binding pocket remain virtually unchanged. However, the Y306A mutation itself results in a considerable enlargement of the ncAA binding pocket. This pocket extends along the α6 helix up to the η1 310-helix that is shielded by Y306 in PylRS_wt and does not take part in pyrrolysine or ncAA recognition in previous crystal structures. PylRS_AF thus offers enhanced potential for the genetic encoding of very large ncAA by providing significantly more space than found in all previously reported structures of wt or mutant PylRS. The Y306A mutation further exposes residues - in particular L407 and D408 - that have been shielded by Y306 in previous crystal structures and now provide a new region for the engineering of further enlarged binding pockets. On the other hand, the found pocket provides sufficient room to accommodate all ncAA tested in the present study and thus solely steric reasons can not account for the observed differences in amber suppression efficiencies with ncAA 3 - 7.

Previous *in vitro* aminoacylation studies with PylRS_wt and amide-substituted lysine-derivatives indicated a strict requirement for the presence of an electronegative heteroatom in the acyl substituent at a similar position as found in pyrrolysine. ncAA bearing this heteroatom are often well tolerated as substrates of PylRS_wt. For example, the use of Nε-carbamate-linkages (that bear an appropriately positionend oxygen atom) to derivatize lysine has been a very successful strategy for the genetic encoding of ncAA with diverse substituents that do not contain further heteroatoms. In contrast, Nε-phenylcarbonyl-L-lysine-and Nε-(pyridyl-3-carbonyl)-L-lysine that share similarity with ncAA 3 - 7 in respect to the amide-connected aromatic ring - but do not bear a heteroatom at a position similar to pyrrolysine - are poor substrates for PylRS_wt *in vitro.* Additionally, all previous crystal structures of PylRS in complex with both Nε-amide- and Nε-carbamate-linked lysine derivatives and AMPPNP or respective AMP-esters showed extensive hydration of the amino acid binding pocket and several hydrogen-bonds between PylRS (in particular N346), water molecules and the side chain of the bound amino acid, i.e. the amid- or carbamate moiety and pyrroline-N-atom. Differences in hydrogenbonding capacity of ncAA 3 - 7 with PylRS_AF due to the number and positions of nitrogen atoms and the presence of an additional hydroxy group are thus a likely origin for the observed differential amber suppression efficiencies.

### Potential of the PylRS_AF Amino Acid Binding Pocket for Further Enlargement.

The ncAA incorporation studies with ncAA3 - 7 provide rules for chromphore design: quinoline- and quinoxalinecarboxamides without further substituents are efficiently processed by PylRS_AF and variations of the fused benzene ring positions in the former are tolerated. Additionally, the solved crystal structure of PylRS_AF shows a significantly larger amino acid binding pocket than found in previous PylRS with available structural data. This provides a basis to further enlarge the binding pocket in order to overcome previous size limitations for aromatic ncAA and genetically encode extended three-ring intercalators derived from quinoline or quinoxaline. The amino acids that are closest to the distal pyrroline ring atoms of pyrrolysine 1 and that were previously known to form part of the rear amino acid binding pocket, L309 and C348, are highly mutable. Moreover, the PylRS_AF structure reveals M276, L407 and D408 as residues that form the rear part of the new, enlarged binding pocket of PylRS_AF (**Figure 6** **A**). M276 has been shown to be mutable, whereas L407 and D408 have previously not been analysed. The inventors performed expressions with *E. coli* that had been cotransformed with plamids pBAD_GFP-39TAG and pREP_PylRS_AF_L407A (including SEQ ID NO.: 5) or pREP_PylRS_AF_D408A (including SEQ ID NO.: 6) (encoding *M*. *mazei* tRNA^{Pyl} and PylRS_AF with the respective additional alanine mutations) in presence of ncAA 2 and 5 and measured cellular GFP expression as described before. With both PylRS mutants, significant levels of GFP-Y39→2 and GFP-Y39→5 were expressed (**Figure 6** **B**). Expression levels were thereby higher for 2 than for 5 with comparable position dependence for both ncAA. This shows that the binding pocket of PylRS_AF is flexible and can be further enlarged and that considerable structural differences in the side chain of the bound ncAA are well tolerated. This provides a promising basis to evolve PylRS mutants for the genetic encoding of ncAA with further extended three-ring chromophores based on the here described quinoline- and quinoxaline-bearing ncAA.

### References

Davidson A, Leeper TC, Athanassiou Z, Patora-Komisarska K, Karn J, Robinson JA, Varani G. Proc Natl Acad Sci U S A. 2009 Jul 21;106(29):11931-6. Simultaneous recognition of HIV-1 TAR RNA bulge and loop sequences by cyclic peptide mimics of Tat protein.
Liu CC, Schultz PG. Annu Rev Biochem. 2010;79:413-44. Adding new chemistries to the genetic code.
Pond CD, Marshall KM, Barrows LR. Mol Cancer Ther. 2006 Mar;5(3):739-45. Identification of a small topoisomerase I-binding peptide that has synergistic antitumor activity with 9-aminocamptothecin.
Yanagisawa T, Ishii R, Fukunaga R, Kobayashi T, Sakamoto K, Yokoyama S. Chem Biol. 2008 Nov 24;15(11):1187-97. Multistep engineering of pyrrolysyl-tRNA synthetase to genetically encode N(epsilon)-(o-azidobenzyloxycarbonyl) lysine for site-specific protein modification.

## Claims

1. A non-canonical amino acid comprising a lysine and an aromatic residue, wherein the aromatic residue is bound to the N-epsilon atom of the lysine and comprises at least two aromatic rings.

2. The non-canonical amino acid of claim 1, wherein the aromatic residue comprises at least three aromatic rings.

3. The non-canonical amino acid of claim 1 or 2, wherein at least one of the aromatic rings comprises at least one heteroatom, preferably nitrogen.

4. The non-canonical amino acid of any of claims 1 to 3, wherein the aromatic residue is selected from the group consisting of quinoline, quinoxaline, isoquinoline, benzoquinoline, phenylquinoline, acridine, aminoacridine, phenazine, phenantridine, carbazole, and benzimidazole.

5. The non-canonical amino acid of any of claims 1 to 4, wherein the aromatic residue is bound to the N-epsilon atom of the lysine by an amide-, carbamate- or urea-bond.

6. A non-canonical amino acid selected from the group consisting of

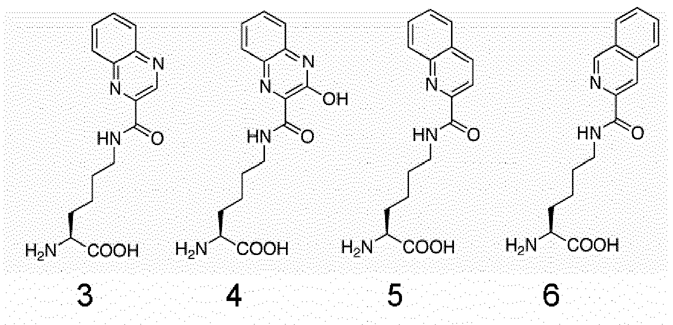

7. A peptide comprising a non-canonical amino acid according to claim 1 or 6.

8. The peptide of claim 7, wherein the peptide is a nucleic acid-binding drug, preferably for treating cancer, bacterial or viral infections.

9. The peptide of any claims 6 to 8, wherein the peptide is bound to a chemical substance, preferably selected from the group consisting of a labeling substance, preferably a fluorescent labeling substance or a radioactive labeling substance, a diagnostic substance, preferably a contrast agent and a pharmaceutical substance, preferably for treating cancer, bacterial infections or viral infections.

10. A method for producing a nucleic acid-binding compound comprising the steps of
- providing a nucleic acid sequence encoding for a peptide,
- introducing a stop codon or a quadruplet codon into the sequence,
- expressing the sequence in the presence of a tRNA^{Pyl}, a modified PylRS, and a non-canonical amino acid according to claim 1, which is encoded by the stop codon or the quadruplet codon,
wherein the aromatic residue is an nucleic acid intercalating residue.

11. The method of claim 10, wherein the sequence is expressed in a microorganism or an *in vitro* translation system, preferably using a modified PylRS.

12. A kit comprising
- a non-canonical amino acid according to claim 1,
- a nucleic acid encoding tRNA^{Pyl}, and
- a nucleic acid encoding a modified PylRS.

13. Use of a non-canonical amino acid comprising a lysine and an aromatic residue for producing a peptide, wherein the aromatic residue is bound to the N-epsilon atom of the lysine and comprises at least two aromatic rings.

14. A modified pyrrolysyl-tRNA-synthetase (PylRS) having a substitution of alanine or glycine for tyrosine at position 306, wherein the PylRS further comprises a substitution of alanine or glycine for lysine at position 407 and/or a substitution of alanine or glycine for aspartic acid at position 408.

15. A nucleic acid encoding for a PylRS according to claim 14.
